**Europäisches Patentamt**

**European Patent Office**

**Office européen des brevets**

(19)

(11) Veröffentlichungsnummer: **0 106 339**

**A2**

(12)

# EUROPÄISCHE PATENTANMELDUNG

(21) Anmeldenummer: **83110273.6**

(22) Anmeldetag: **14.10.83**

(51) Int. Cl.³: **G 01 N 21/64**
**G 01 N 15/07, G 01 N 1/30**

(30) Priorität: **15.10.82 DE 3238353**

(43) Veröffentlichungstag der Anmeldung:
**25.04.84 Patentblatt 84/17**

(84) Benannte Vertragsstaaten:
**AT BE CH DE FR GB IT LI LU NL SE**

(71) Anmelder: **Max-Planck-Gesellschaft zur Förderung der Wissenschaften e.V.**
**Bunsenstrasse 10**
**D-3400 Göttingen(DE)**

(72) Erfinder: **Valet, Günther, Prof.Dr.**
**Gräfelfingerstrasse 79a**
**D-8000 München(DE)**

(74) Vertreter: **Weickmann, Heinrich, Dipl.-Ing. et al,**
**Patentanwälte Dipl.-Ing. H.Weickmann Dipl.-Phys.Dr.**
**K.Fincke Dipl.-Ing. F.A.Weickmann Dipl.-Chem. B. Huber**
**Dr.-Ing. H. Liska Dipl.-Phys.Dr. J. Prechtel Postfach**
**860820**
**D-8000 München 86(DE)**

(54) **Verfahren zur simultanen quantitativen Bestimmung der Zellen und Reagenz hierfür.**

(57) Zur simultanen quantitativen Bestimmung der Blutzellen inkubiert man die zu bestimmende Blutprobe mit einem Fluoreszenzfarbstoff, der wenigstens eine Eigenschaft der Blutzellen anfärbt und mißt danach das Volumen und die Fluoreszenz der Zellen bei mindestens einer Wellenlänge gleichzeitig.

Durchflußcytometer Schemadarstellung

FIG.1

EP 0 106 339 A2

Beschreibung

Die Erfindung betrifft ein Verfahren zur simultanen quantitativen Bestimmung der Zellen und ein zu seiner Durchführung geeignetes Reagenz.

Die Blutzellenzählung ist eine der am häufigsten angeordneten Laboruntersuchungen im Klinik- und Praxisbereich. Man schätzt, daß allein in der Bundesrepublik Deutschland täglich zehntausend derartige Bestimmungen durchgeführt werden. Die Bestimmung ist von besonderer Bedeutung im Rahmen der Unfallmedizin, der Intensivmedizin und für den Operationssaal.

Für ein vollständiges Blutbild werden üblicherweise sechs separate Untersuchungen aus jeder Blutprobe durchgeführt:

| | | |
|---|---|---|
| 1. | Erythrozytenzählung | (elektrischer Zähler, Zählkammer) |
| 2. | Leukozytenzählung | (elektrischer Zähler, Zählkammer) |
| 3. | Thrombozytenzählung | (elektrischer oder Streulichtzähler, Zählkammer) |
| 4. | Hämatokrit | (Zentrifuge) zur Berechnung des mittleren Erythrozytenvolumens (MCV) |
| 5. | Retikulozyten | (Anfärbung mit Brillantkresylblau, Ausstrich und Zählung) |
| 6. | Leukozyten-differenzierung | (Ausstrich, May-Grünwald-Giemsa Färbung und Auszählung) |

Vor allem die Retikulozyten- und Thrombozytenzählung sowie das Differentialblutbild sind zeitaufwendig oder methodisch schwierig. So ist der Zeitaufwand für eine quantitative Bestimmung der Blutzellen in Stunden zu messen. Gleiches gilt auch für die Bestimmung anderer Zellen, die als Einzelzellen vorliegen, beispielsweise durch mechanische oder chemische Desintegration von Gewebe. Daher besteht ein Bedürfnis nach der Entwicklung schneller und automatisierbarer Verfahren, die es erlauben, den bisher erforderlichen großen Zeit- und Personalaufwand wesentlich zu verringern.

Diese Aufgabe wird erfindungsgemäß gelöst durch ein Verfahren zur simultanen quantitativen Bestimmung von Zellen, welches dadurch gekennzeichnet ist, daß es die zu bestimmende Zellen enthaltende Probe mit einem Fluoreszenzfarbstoff, der wenigstens eine Eigenschaft der Blutzellen anfärbt, inkubiert und danach das Volumen und die Fluoreszenz der Zellen bei mindestens einer Wellenlänge gleichzeitig mißt.

Das Verfahren der Erfindung beruht im Prinzip darauf, daß Eigenschaften der Zellen durch Farbstoffe angefärbt und gleichzeitig mit dem Zellvolumen gemessen werden, beispielsweise in einem Durchflußcytometer. Durch die gleichzeitige Bestimmung des Volumens und der Fluoreszenz der angefärbten Zellen, die im Automaten durchgeführt wird, wird jede einzelne Zelle nach Art und Menge erfaßt und kann je nach bestimmtem Zellvolumen und Fluoreszenz einer bestimmten Blutzellart zugeordnet werden, da jede der Zellarten durch bestimmte Werte von Fluoreszenz und Zellvolumen gekennzeichnet ist. Auf diese Weise wird es z. B. möglich, innerhalb weniger Minuten ein vollständiges Blutbild mit minimalem Personalaufwand zu erstellen.

Die durch die erfindungsgemäß eingesetzten Farbstoffe angefärbten Eigenschaften der Zellen ｜sind Bestands- oder Funktionseigenschaften. Bestandseigenschaften sind solche, die durch celluläre Synthese entstehen, wie z. B. DNS, RNS, Proteine und Lipide, während Funktionseigenschaften Resultanten aus Stoffwechselprozessen sind wie z. B. Transmembranpotential und intracellulärer pH-Wert.

Als besonders geeignet für das erfindungsgemäße Verfahren hat sich die gleichzeitige Anfärbung der Zellen mit einem DNS/RNS-Farbstoff und einem membranpotentialsensitiven Farbstoff erwiesen und wird daher bevorzugt. Für diese bevorzugte Ausführungsform der Erfindung besonders geeignete DNS/RNS-Farbstoffe sind Acridinorange (AO), Chinacrin (Quinacrin) und Pyronin Y, wobei ersteres besonders bevorzugt wird. Aus der Gruppe der membranpotentialsensitiven Farbstoffe wird das 3,3'-Dihexyl-oxa-carboxyanin (DiOC6(3)) bevorzugt. Die bevorzugten Substanzen sind im selben Spektralbereich mit derselben Lichtquelle anregbar und eignen sich daher zur Herstellung eines vorgemischten Reagenz, welches den Zellproben zugesetzt werden kann.

Beispiele für andere im Rahmen der Erfindung geeignete Farbstoffe aus der oben erwähnten Gruppe sind Fluoresz- amin (Fluram), 1-Anilinonaphtalin-8-sulfonsäure (ANS) und o-Phthalaldehyd für die Anfärbung des Zellproteins, 4-Aminoacridin zur Anfärbung der Lipide, N-(3-Fluo- anthyl)maleimid zur Anfärbung freier SH-Gruppen, Fluoresceindiacetat (FDA) zur Anfärbung von Enzymaktivitäten (im genannten Fall der Esteraktivität) und 1,4-Di- acetoxy-2,3-Dicyano-Benzol (ADB) zur Anfärbung des intracellulären pH-Wertes.

Das Volumen bzw. die Größenverteilung der Zellen wird
entweder durch Lichtstreuung (J. Histochem. Cytochem.
27, 359-365 (1979)) oder durch Messung der elektrischen
Widerstandsveränderung beim Passieren eines engen
Durchlasses (Coulter-Verfahren, vgl. z. B. "Flow cytometry and sorting, von Melamed, Mullaney und Mendelsohn,
John Wiley and sons, Inc. 1979, Seiten 61 bis 101) bestimmt. Das Coulter-Verfahren wird bevorzugt. Es beruht
darauf, daß die Blutprobe durch eine kurze Öffnung geringen Durchmessers laufen gelassen wird und an dieser
Stelle die Änderung des elektrischen Widerstandes
gemessen wird, wobei der Teilchenwiderstand sich von
dem des Elektrolyten unterscheidet. Die Spannungsänderung, die beim Durchgang einer Zelle durch die Öffnung,
über die zwischen zwei Elektroden ein konstanter elektrischer Strom angelegt wird, auftritt, ist dem Teilchenvolumen direkt proportional.

Die Inkubation mit dem Farbstoff läßt sich bequem bei
Zimmertemperatur innerhalb weniger Minuten durchführen.
Im allgemeinen reichen 1 bis 10, vorzugsweise 2 bis 6
Minuten Stehenlassen bei Zimmertemperatur aus, gerechnet
ab Zugabe einer Lösung der Farbstoffe zu der zu untersuchenden Blutprobe, die zweckmäßig mit isotoner Kochsalzlösung geeignet verdünnt wurde. Nach der Inkubation
gibt man die zu untersuchende Probe in eine geeignete
Apparatur, beispielsweise einen handelsüblichen Durchflußcytometer, der so eingerichtet sein muß, daß das
Zellvolumen und die Fluoreszenz gleichzeitig bestimmt
werden können und damit dem für jedes Teilchen gemessenen Volumen auch eine entsprechende Fluoreszenzmessung
zugeordnet werden kann.

Die elektrische Methode wird bevorzugt, da die experimentiell bestimmten Farbstoffgehalte der Einzelzellen jeder Blutzellart als Farbstoffkonzentrationen ausgedrückt werden können, da Absolutvolumina gemessen werden. Dadurch können die gemessenen Eigenschaften der Zellarten in normalisierter Weise direkt miteinander verglichen werden. Bei der Lichtstreumethode hingegen ist dies nicht möglich, da die Lichtstreuung neben Zellvolumen und Form noch von der Beschaffenheit von Zelloberfläche und Zellinnerem abhängt.

Bei der bevorzugten Ausführungsform der Erfindung unter Verwendung von Acridinorange (AO) in Kombination mit DiOC6 macht man sich zunutze, daß durch AO alle Blutzellen, ausgenommen die Erythrocyten, gut angefärbt werden, durch DiOC6 auch die Erythrocyten gut gefärbt werden, gleichzeitig jedoch die Färbung der übrigen Zellen verbessert wird.

Gemäß einer weiteren bevorzugten Ausführungsform der Erfindung wird die Messung in Gegenwart einer fluoreszierenden, insbesondere einer mit einem Fluoreszenzfarbstoff gefärbten monodispersen Eichphase durchgeführt. Bei bekannter Konzentration der zugegebenen fluoreszierenden Eichphase wird eine Bestimmung der Absolutkonzentrationen der verschiedenen Zellen im Blut möglich. Als Eichphase werden bevorzugt monodisperse Latexpartikel mit einem Durchmesser von 1 bis 10 µm, besonders bevorzugt zwischen 4 und 6 µm, verwendet. Jedoch können auch andere fluoreszierende Partikel bekannter einheitlicher Größe im Bereich der Blutzellen und bekannter Konzentration verwendet werden.

Die Durchführung des erfindungsgemäßen Verfahrens ist äußerst einfach. Blut wird mit physiologischer Kochsalzlösung und dem Farbstoff sowie gegebenenfalls den Partikeln der Eichphase versetzt. Nach beispielsweise drei bis fünf Minuten Anfärbezeit werden die gefärbten Blutzellen in einem geeigneten Gerät gemessen, beispielsweise unter Verwendung eines handelsüblichen Durchflußcytometers mit einer Geschwindigkeit von etwa 2000 Zellen pro Sekunde während eines Zeitraums von etwa 5 bis 15 Minuten. Auf diese Weise lassen sich alle Arten von Blutzellen in genügender Menge quantitativ erfassen.

Sollen Gewebszellen bestimmt werden, so werden diese zuerst durch Desintegration des entsprechenden Gewebes z. B. durch Zerschneiden mit einem "tissue chopper" in Freiheit gesetzt! Das weitere Verfahren entspricht dann dem oben für Blutzellen beschriebenen.

Das Verfahren der Erfindung ermöglicht es weiterhin, nicht nur eine sondern mehrere Fluoreszenzen zu messen. Beispielsweise kann bei der bevorzugten Ausführung des Verfahrens unter Verwendung von AO und DiOC6 sowohl die Gelbfluoreszenz nativer DNS/RNS (spiralisierte Form) als auch die Rotfluoreszenz der entspiralisierten Form bestimmt werden. Hierdurch wird es möglich, zusätzlich eine Aussage über den Funktionszustand der Blutzellen zu machen. Das Verfahren der Erfindung ermöglicht es in dieser Ausführungsform daher, nicht nur eine quantitative Blutzellzählung durchzuführen, sondern auch noch Aussagen über den Funktionszustand der bestimmten einzelnen Zellarten zu machen, was mit bekannten Verfahren nicht möglich ist.

Die erfindungsgemäß verwendeten Farbstoffe lassen sich sowohl mit optischen Systemen bestimmen, die mit Quecksilber- bzw. Xenonlampen ausgerüstet sind als auch mit solchen, die mit Lasereinrichtungen versehen sind.

Ein weiterer Gegenstand der Erfindung ist ein Reagenz zur Durchführung des erfindungsgemäßen Verfahrens, welches dadurch gekennzeichnet ist, daß es einen Fluoreszenzfarbstoff aus der Gruppe DNS/RNS-Farbstoffe, Zellprotein-Farbstoffe, Lipid-Farbstoffe, Enzym-Farbstoffe, Membranpotentialsensitive-Farbstoffe, Intracellulär-pH-Farbstoffe, SH-Gruppen-Farbstoffe und zusätzlich eine monodisperse Eichphase enthält.

Ein bevorzugtes Reagenz gemäß der Erfindung enthält einen DNS/RNS-Farbstoff und einen Membranpotentialsensitiven-Farbstoff.

Besonders bevorzugt wird ein Reagenz, welches Acridinorange, 3,3'-Dihexyl-oxy-carbocyanin, monodisperse Latexpartikel mit 1 bis 10 µ Durchmesser und ein Lösungsmittel enthält.

Geeignete Lösungsmittel für das erfindungsgemäße Reagenz sind solche, welche den jeweils gewählten Farbstoff und die monodisperse Phase in ausreichender Konzentration in Lösung bringen können, ohne die Partikel der Eichphase anzugreifen, beispielsweise anzulösen. Bevorzugte Lösungsmittel sind Dimethylsulfoxyd, Dimethylformamid und Alkanole.

Durch die Erfindung wird erreicht, daß simultan alle Zellarten in wenigen Minuten quantitativ bestimmt werden können, wobei die schnelle Färbezeit und die vollständige Automatisierbarkeit des Verfahrens, nämlich Färbung, Messung und Auswertung, hieran entscheidenden Anteil haben. Dabei ist es möglich, neben der Zellzählung außerdem auch noch Aussagen über den Funktionszustand der einzelnen Zellarten zu erhalten.

Darüberhinaus ermöglicht das Verfahren nicht nur eine Verkürzung der Meßzeiten und eine Erweiterung der Aussagekraft sondern kann auch in einem besonders großen Meßbereich angewendet werden, der etwa 2,5 Dekaden log, d. h. etwa 1 : 500 beträgt.

Die Bedeutung dieses Vorteils läßt sich aus folgendem erkennen: Geht man von den Normalkonzentrationen der verschiedenen Blutzellen aus, die bei Erythrocyten bei $5 \times 10^6$, bei Thrombocyten bei $3 \times 10^5$, bei Leukocyten bei $5 \times 10^3$ pro mm³ liegen, so ist erkennbar, daß ein breiter Meßbereich unbedingt erforderlich ist, wenn alle diese Blutzellen simultan bestimmt werden sollen. So war es bisher schwierig, Thrombocyten-Konzentrationen unter 5 bis 7 $\times 10^5$ pro mm³ zu bestimmen. Erfindungsgemäß wird diese Untergrenze auf $1 \times 10^3$ pro mm³ heruntergesetzt. Dies ist deshalb von Bedeutung, da gerade im Bereich von 100.000 und um 30.000 pro mm³ die besonders kritischen Bereiche liegen. Im ersteren Fall ist dies der pathologische Bereich, im letzteren Fall der akut gefährliche Bereich.

Eine weitere Anwendungsmöglichkeit des erfindungsgemäßen Verfahrens besteht in der Untersuchung der Wirkung von Medikamenten auf einzelne Zellen, beispielsweise der Wirkung cytostatischer Medikamente auf Tumorzellen. Dies ermöglicht insbesondere bei Medikamenten mit hoher Toxizität eine Vorabtestung ob ihre Wirksamkeit beim speziellen Individuum die Inkaufnahme der toxischen Nebenwirkungen rechtfertigt oder nicht. Beispielsweise können aus Tumorgewebe Einzelzellen mechanisch gewonnen, in einem geeigneten Nährmedium, beispielsweise in haparinisiertem Patientenblutplasma, in Gegenwart des zu untersuchenden Medikaments getestet und anschließend quantitativ nach dem erfindungsgemäßen Verfahren bestimmt werden, welcher Anteil der Tumorzellen durch das Medikament abgetötet wurde und welcher Anteil noch lebt. Das Verfahren der Erfindung läßt sich so dazu benutzen, das jeweils wirksamste unter mehreren in betracht kommenden Medikamenten herauszufinden.

Die folgenden Beispiele erläutern die Erfindung in Verbindung mit der Zeichnung weiter. In dieser stellen dar:

Fig. 1     eine schematische Darstellung eines für die
           Durchführung der Erfindung geeigneten Durchflußcytometers,

Fig. 2     die graphische Darstellung von Zellvolumen
           gegen Fluoreszenz einer gefärbten (a) und
           einer ungefärbten (b) 1 : 250 verdünnten Blutprobe, (Th=Thrombozyten, Er=Erythrozyten, Re=Retikulo-
           zyten, St=Eichpartikel, Ly=Lymphozyten, Gr=Granulo-
           zyten, Ba=Basislinie)

Fig. 3     eine graphische Darstellung, wie in Fig. 2
           unter Verwendung anderer Farbstoffe,

Fig. 4     eine graphische Darstellung analog Fig. 2
           unter Verwendung wiederum anderer Farbstoffe,

Fig. 5     eine graphische Darstellung bei simultaner
           Messung von 3 Parametern, wobei neben dem
           Zellvolumen 2 Fluoreszenzen aufgetragen sind.

Fig. 6     eine graphische Auftragung der im Rahmen der
           3-Parameter-Messung von Fig. 5 erhaltenen
           beiden Fluoreszenzmessungen gegeneinander, so
           daß die Stoffwechselzustände der Zellen
           daraus entnommen werden können.

Fig. 7    eine graphische Darstellung der Ergebnisse
          einer Medikamentenaustestung bei Zellen
          desintegrierter Lymphknotenmetastasen eines
          Mammakarzinoms, wobei die Art des Medikaments
          gegen die Zahl der Tumorzellen bzw. Entzündungszellen aufgetragen ist.


B e i s p i e l    1


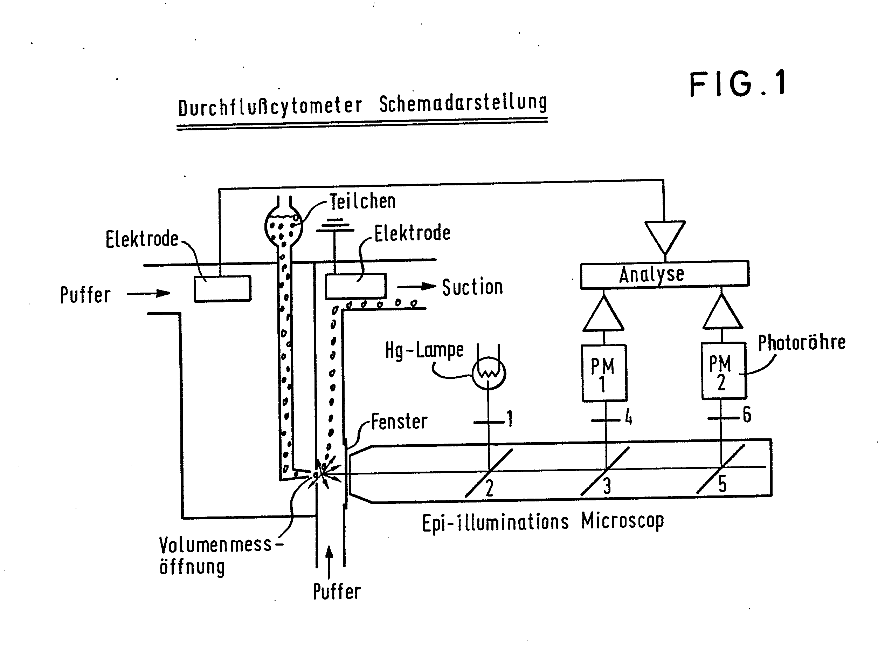

5 µl Blut werden aus der Fingerbeere entnommen und 1/250
mit isotoner Kochsalzlösung (0,15 M NaCl mit 10 mM
TRIS/HCL pH 7,4, (TBS)) verdünnt. 500 µl der Zellsuspension
werden mit 5 µl Reagenz (AO 0,4 mg/ml, DiOC6 0,02 µg/ml
und 4 µm monodisperse, mit FITC gefärbte Latexpartikel
der Konzentration $5 \times 10^7$/ml) während 3 bis 5 Minuten
bei Zimmertemperatur inkubiert. Als Lösungs- und Suspensionsmedium für das Reagenz wird Dimethylsulfoxyd
(DMSO) verwendet. Die Suspension wird durch Schütteln
gut durchgemischt und zur Messung in ein Durchflußcytometer eingebracht, dessen Bauprinzip in Fig. 1 der beigefügten Zeichnung dargestellt ist. Das Gerät ist im Handel erhältlich unter der Bezeichnung Fluvo-Metricell.
Im Falle der Färbung mit AO/DiOC6 werden folgende Filter und Spiegel verwendet:

1  Kurzpaß (KP) 500 nm mit Langpaß (LP) 418 nm Filter;
2  Dichroitischer Teilerspiegel (D) 500 nm;
3  Reflexionsspiegel bei Zweiparameter-Messung bzw.
   D 530 nm bei Dreiparameter-Messung;
4  LP 500 nm;
5  Reflexionsspiegel;
6  LP 550 nm.

AO/DiOC6 werden zwischen 418 und 500 nm angeregt. Das emittierte Fluoroeszenzlicht wird bei Zweiparameter-Messungen (Volumen gegen Fluoroeszenz 1) zwischen 500 und 700 nm durch die Photoröhre 1 (PM1) gesammelt. Bei Dreiparameter-Messungen werden das gelbe Licht zwischen 500 und 530 nm und das rote Licht zwischen 550 und 700 nm durch PM1 und PM2 gemessen.

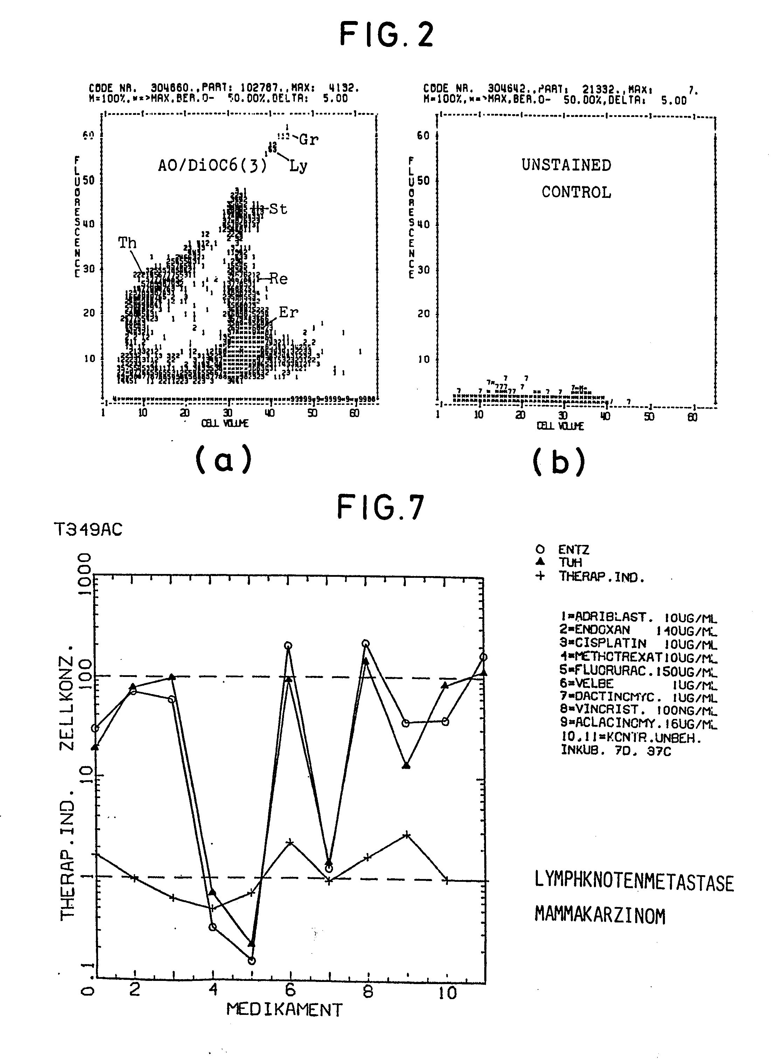

Für die in den Fig. 2 bis 6 der Zeichnung dargestellten Ergebnisse wurde mit einer zylindrischen Meßöffnung von 50 μm Durchmesser und 50 μm Länge gearbeitet. Das Flüssigkeitssystem des Durchflußcytometers war mit TBS-Puffer 25° C gefüllt. Das Zellvolumen wurde bei einem elektrischen Strom von 0,385 mA gemessen.

Die Fluoreszenz wurde mit einer HBO-100 Hg Hochdrucklampe erregt. Die logaritmisch verstärkten Fluoreszenz- und Volumensignale der Zellen wurden entweder in einem Vielkanalanalysator als Zweiparameter-Histogramme gespeichert oder ON-LINE auf ein Magnetband geschrieben. Von Magnetband wurden die Kurven graphisch und rechnerisch ausgewertet. Bei Verwendung eines mikroprozessorgesteuerten Datenmoduls können sowohl Farbstoffzugabe und Messung, als auch die Auswertung im Prinzip vollständig automatisiert werden.

Fig. 2a zeigt in graphischer Darstellung Zellvolumen gegen Fluoreszenz aufgetragen.

Die Ausrechnung durch Integration der Zellhaufen in Abb. 2a ergibt folgende Werte

| | Thrombozyten | Erythrozyten | Retikulozyten | Eichpartikel | Lymphozyten | Granulozyten | |
|---|---|---|---|---|---|---|---|
| Anteil | 4.6o | 91.62 | 1.62 | 2.02 | .o24 | .o45 | % |
| Konz.in d. Meßprobe | 11.38 | 226.7 | 4.18 | 5.oo | .o594 | .111 | $\times 10^5$/ml |
| Konz.im Blut | 2845oo | $5.66 \times 10^6$ | 1o45oo | - | 1485 | 275o | Zellen/ ml |

Fig. 2b zeigt das Ergebnis einer analogen Messung ohne Zusatz von Farbstoff. Es ist keine zellgebundene Fluoreszenz feststellbar.

Die Volumen- und Fluoroeszenzpulse des FLUVO-METRICELL-Durchflußcytometers wurden mit Hilfe von 2,5 Dekaden logarithmischen Verstärkern logarithmiert und danach entsprechend ihrer maximalen Amplitude in die 64 x 64 Matrix eines Vielkanalanalysators eingezählt. Zur graphischen Darstellung wurden die Kanalinhalte der Matrix logarithmiert (3 Dekaden Amplituden-Log) und auf den mit (M) bezeichneten Wert normiert. Der maximale Kanalinhalt (M) wurde in 20 gleiche Teile geteilt (5% Schritte). Jeder Kanalinhalt erhielt eine Zahl zwischen 1 bis 10 entsprechend seiner relativen Häufigkeit. Kanalinhalte, deren Amplitude höher als 50% des Maximalwertes war, wurden durch (*) gekennzeichnet.

B e i s p i e l e    2 bis 9

Wie in Beispiel 1 beschrieben, wurde unter Verwendung der gleichen Apparatur die simultane quantitative Bestimmung der Blutzellen bei je 500 µl 1 : 250 verdünntem Humanblut durchgeführt, welches jeweils mit 5 µl Reagenz gemäß nachstehender Tabelle gefärbt worden war:

| Beispiel | Farbstoff | Zusammensetzung | Figur |
|----------|-----------|-----------------|-------|
| 2 | Quinacrin | 0,2 mg/ml DMSO | 3a |
| 3 | Fluram | 1,0 mg/ml DMSO | 3b |
| 4 | ANS | 2,0 mg/ml DMSO | 3c |
| 5 | o-Phthaldehyd | 3,0 mg/ml DMSO | 3d |
| 6 | 4-Aminoacridin | 1,0 mg/ml Äthanol | 4a |
| 7 | F-Maleimid | 1,0 mg/ml DMSO | 4b |
| 8 | FDA | 0,04 mg/ml Dimethyl-Formamid | 4c |
| 9 | ADB | 1,0 mg/ml Dimethyl-Formamid | 4d |

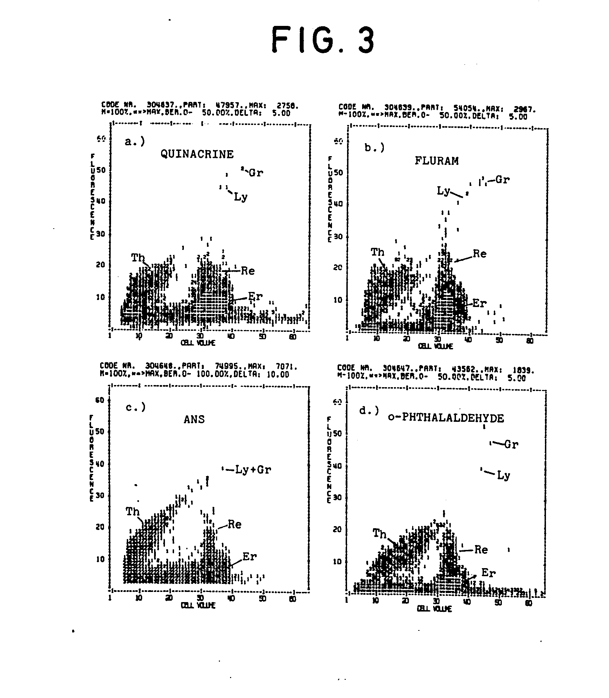

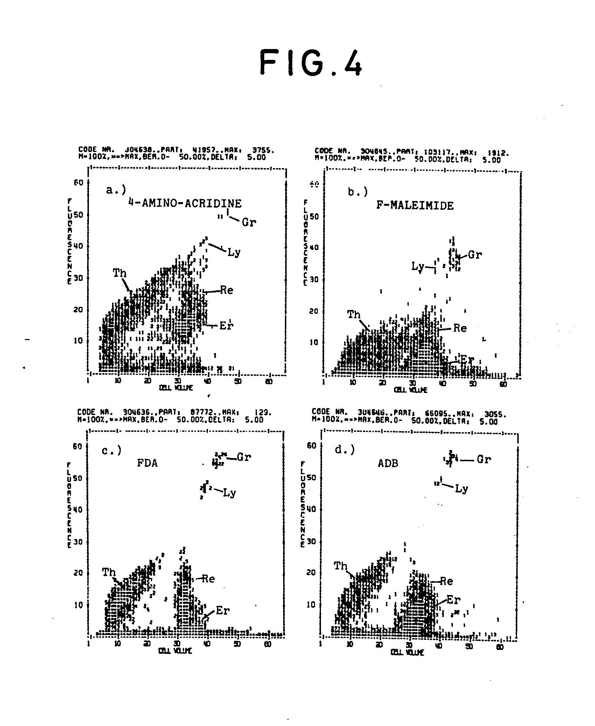

Die Ergebnisse sind in Fig. 3 und 4 graphisch dargestellt

0106339

Beispiel 10

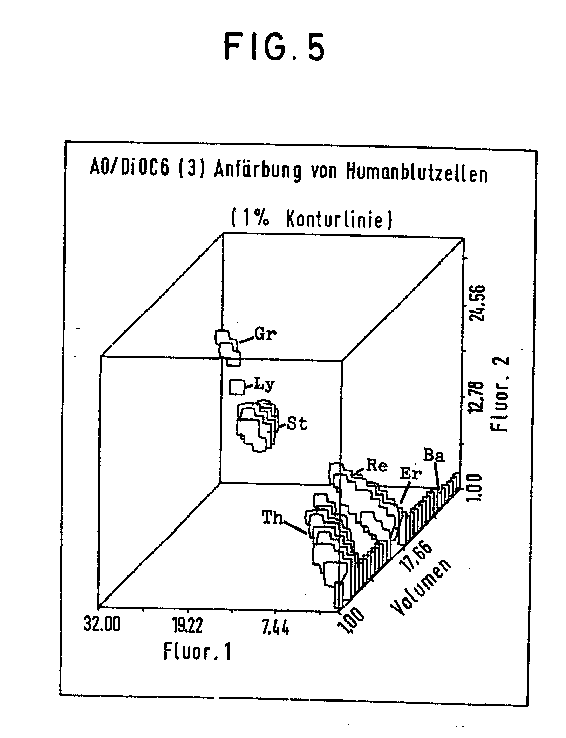

Wie im Beispiel 1 beschrieben, wurde eine Bestimmung
bei einer mit AO/DiOC6 gefärbten Blutprobe durchgeführt,
jedoch unter Aufzeichnung von zwei verschiedenen
Fluoreszenzen und zwar der gelben (Fluoreszenz 1;
Transmembranpotential bei der AO/DiOC6-Färbung) und der
roten (Fluoreszenz 2; entspiralisierte RNS/DNS). Die
Erfassung dieser drei Parameter ermöglicht die in Fig. 5
dargestellte Wiedergabe der Meßdaten in Wolkenform.

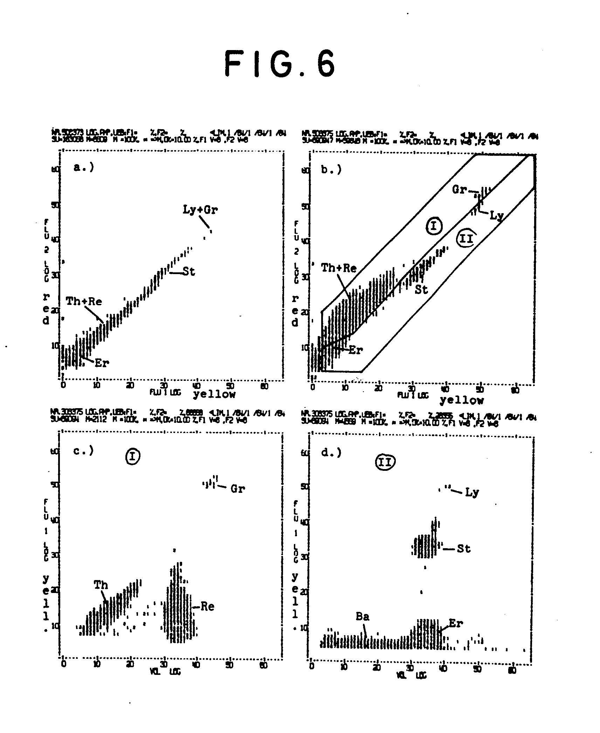

Jede Skala in Fig. 5 und 6 umfaßt 2,5 logarithmische
Dekaden. Die drei Meßpulse jeder einzelnen Zelle wurden
für die Darstellung in Fig. 5 logarithmiert und ON-LINE
auf Magnetband übertragen. Zur Auswertung wurden die
Werte in einer 32 x 32 x 32 Matrix klassiert und mit
Hilfe eines Wolkenprogramms (. Cytometry
1., 222 :-228 (1980)) dargestellt. Das Vorhandensein von
Zellen und Eichpartikeln ist durch eine Konturlinie bei
1 % des maximalen Kanalinhalts wiedergegeben. Die einzelnen Zellarten und die Eichpartikel sind gut voneinander
unterschieden.

Fig. 6 gibt eine Darstellung der gelben (Fluor. 1)
gegen die rote (Fluor. 2) Fluoreszenz von 3-Parametermessungen, bei denen 1/500 verdünntes Humanblut mit
DiOC6 (a) und mit AO/DiOC6 gleichzeitig (b) gefärbt
worden war. Wie man erkennt, ermöglicht es die Dreiparametermessung, RNS-Gehalt und RNS-Konzentration der
verschiedenen Zelltypen zu bestimmen. Durch die zusätzlich AO-Färbung erhält der mit Th+Re gekennzeichnete
Teil der Kurve 6b eine deutliche Rotverschiebung. Werden die Zellvolumina gegen Fluoreszenz 1 Histogramme
der rotverschobenen (I) und der unverändert gebliebenen (II) Partikel gezeichnet (c,d), so zeigt der Vergleich mit Fig. 1a daß die RNS haltigen roten Zellen den Thrombocyten (Th), den Retikulocyten (Re) und den Granulocyten (Gr) entsprechen, während die Erythrocyten (Er), die Eichpartikel (St) und die Lymphozyten (Ly) keine RNS enthalten und deshalb ihre Gelbfluoreszenz beibehalten haben. Die Doppelfärbung zeigt als Nebenergebnis auch, daß die Retikulozyten ein höheres Membranpotential aufweisen als die Erythrocyten. Dies läßt sich aus den Fig. 6a und 6b entnehmen. Die direkt über dem Erythrocytenhaufen liegenden Zellen entsprechen der in Fig. 6a mit Th+Re gekennzeichneten Zone. Diese Zone wird durch die AO-Zusatzfärbung (6b) quantitativ ins Rote verschoben, was bedeutet, daß die durch DiOC6 gelb gefärbten Zellen RNS enthalten. Im Volumen/Fluoreszenz-Histogramm entsprechen diese Zellen den Thromocyten und Retikulocyten. Dies zeigt, daß die über den Erythrocyten liegenden mit DiOC6 gefärbten Zellen Retikulocyten sind.

- 19 -

0106339

Beispiel 11

Frisches steriles Material einer Lymphknotenmetastase eines Mammakarzinoms wird mechanisch mit einer Gewebszerkleinerungsvorrichtung (tissue chopper) in Stücke zerschnitten, die durch ein Sieb mit 60 µm lichter Maschenweite gesiebt werden. Die erhaltenen Zellen werden sieben Tage in heparinisiertem Patientenblutplasma als Kulturmedium in Mikrotiterplatten in Gegenwart oder Abwesenheit verschiedener Cytostatika kultiviert. Dann wird die Zellsuspension gewaschen und fünf Minuten mit 1,4-Diacetoxy-2,3-Dicyano-Benzol (ADB) und Propidiumjodid (PI) gefärbt. ADB zeigt die Aktivität der Cytoplasmaesterase und den intracellulären pH-Wert der lebenden Zellen an, PI färbt die DNA abgestorbener Zellen. Das Zellvolumen und die blaue und die grüne Fluoreszenz der gefärbten Zellen werden gleichzeitig in einem Durchflußcytometer, wie im Beispiel 1 beschrieben, gemessen. Vor der Messung werden der Zellsuspension als Konzentrations- und Fluoreszenzstandard fluoreszierende monodisperse Latexteilchen von 6 µm zugesetzt. Zellvolumen und die Fluoreszenzsignale jeder Zelle, welche pH-Wert, Esteraseaktivität und DNS entsprechen, werden simultan gemessen.

Die Zahl der in einer Kultur lebenden bzw. überlebenden Tumorzellen oder Entzündungszellen wird dann aus ihrem Verhältnis zur standardisierten Eichpartikelzahl berechnet. Die Ergebnisse der verschiedenen untersuchten Cytostatika und der unbehandelten Kontrollen sind in dem in Fig. 7 gezeigten Resistenzdiagramm zusammengefaßt. Die Abszisse des Diagramms identifiziert die einzelnen Medikamente und die Kontrollen, auf der Ordinate ist die jeweilige Zahl der Tumorzellen bzw.
Entzündungszellen, bezogen auf 100 % der Kontrollen,
aufgetragen. Man erkennt, daß bei den Cytostatika Nr.
1, 4, 5 und 7 eine Reduktion der Tumorzellen erzielt
wurde, während die übrigen Medikamente keine Wirkung
ergaben. Gesondert ist die Wirkung auf Entzündungszellen erfaßt, welche die weniger toxischen Medikamente
besser erkennen läßt. Das Verhältnis von verbliebenen
Entzündungszellen zu Tumorzellen nach der Inkubation
wird als therapeutischer Index ebenfalls in der Figur
dargestellt.

<u>P a t e n t a n s p r ü c h e</u>

1. Verfahren zur simultanen quantitativen Bestimmung von Zellen, d a d u r c h g e k e n n z e i c h n e t , daß man die zu bestimmenden Zellen enthaltende Probe mit einem Fluoreszenzfarbstoff, der wenigstens eine Eigenschaft der Zellen anfärbt, inkubiert und danach das Volumen und die Fluoreszenz der Zellen bei mindestens einer Wellenlänge gleichzeitig mißt.

2. Verfahren nach Anspruch 1, d a d u r c h g e k e n n z e i c h n e t , daß man wenigstens einen Farbstoff aus der Gruppe DNS/RNS-Farbstoffe, Zellprotein-Farbstoffe, Lipid-Farbstoffe, Enzym-Farbstoffe, Membranpotentialsensitive-Farbstoffe, Intracellulär-pH-Farbstoffe und SH-Gruppen-Farbstoffe verwendet.

3. Verfahren nach Anspruch 2, d a d u r c h g e k e n n z e i c h n e t , daß man sowohl mit einem DNS/RNS Farbstoff als auch mit einem Membranpotential-sensitiven Farbstoff inkubiert.

4. Verfahren nach Anspruch 3, d a d u r c h g e k e n n z e i c h n e t , daß man einen DNS/RNS Farbstoff aus der Gruppe Acridinorange, Chinacrin oder Pyronin Y verwendet.

5. Verfahren nach Anspruch 3 oder 4, d a d u r c h g e k e n n z e i c h n e t , daß man als Membransensitiven Farbstoff 3,3'-Dihexyl-oxa-carbocyanin verwendet.

6. Verfahren nach einem der Ansprüche 1 bis 5, d a d u r c h     g e k e n n z e i c h n e t , daß man die Messung in Gegenwart einer fluoreszierenden monodispersen Eichphase in der Probe durchführt.

7. Verfahren nach Anspruch 6,     d a d u r c h g e k e n n z e i c h n e t ,     daß man als Eichphase monodisperse Latexpartikel von 1 bis 10 µm Durchmesser verwendet.

8. Verfahren nach einem der vorhergehenden Ansprüche, d a d u r c h     g e k e n n z e i c h n e t , daß man die Zellfluoreszenz durch Bestrahlung der strömenden angefärbten Blutprobe beim Passieren eines engen Querschnitts mit gepulstem monochromatischen Licht, Laserlicht oder einer Quecksilber- bzw. Xenonlampe und Messung der erzeugten Fluoreszenzsignale bestimmt.

9. Verfahren nach einem der vorhergehenden Ansprüche, d a d u r c h     g e k e n n z e i c h n e t , daß man das Zellvolumen durch Messung der Änderung der elektrischen Leitfähigkeit der strömenden Probe beim Passieren eines engen Querschnitts bestimmt.

10. Verfahren nach einem der Ansprüche 1 bis 8, d a d u r c h     g e k e n n z e i c h n e t , daß man das Zellvolumen durch Messung der Lichtstreuung der strömenden Probe beim Passieren eines engen Querschnitts bestimmt.

11. Verfahren nach einem der vorhergehenden Ansprüche, d a d u r c h   g e k e n n z e i c h n e t , daß man die Messung in einem Durchflußcytometer oder Zellsorter vornimmt.

12. Reagenz zur Durchführung des Verfahrens nach Anspruch 1 bis 11,   d a d u r c h   g e k e n n z e i c h n e t ,   daß es einen Fluoreszenzfarbstoff aus der Gruppe DNS/RNS-Farbstoffe, Zellprotein-Farbstoffe, Lipid-Farbstoffe, Enzym-Farbstoffe, Membranpotentialsensitive-Farbstoffe, Intracellulär-pH-Farbstoffe, SH-Gruppen-Farbstoffe und zusätzlich eine monodisperse fluoreszierende Eichphase enthält.

13. Reagenz nach Anspruch 12,   d a d u r c h   g e k e n n z e i c h n e t ,   daß es einen DNS/RNS Farbstoff und einen membranpotentialsensitiven Farbstoff enthält.

14. Reagenz nach Anspruch 12 oder 13,   d a d u r c h   g e k e n n z e i c h n e t ,   daß es als monodisperse Eichphase Latexpartikel mit 1 bis 10 µm Durchmesser enthält.

15. Reagenz nach Anspruch 13 und 14,   d a d u r c h   g e k e n n z e i c h n e t ,   daß es Acridin-orange, 3,3'-Dihexyl-oxa-carbocyanin und ein Lösungsmittel enthält.

16. Reagenz nach Anspruch 15,   d a d u r c h   g e k e n n z e i c h n e t ,   daß es als Lösungsmittel Dimethylsulfoxid, Dimethylformamid oder ein Alkanol enthält.

FIG.1

Durchflußcytometer Schemadarstellung

Epi-illuminations Microscop

0106339

2/6

FIG. 2

CODE NR. 304660.,PART: 102787.,MAX: 4192.
M=100%,*=>MAX.BER.0- 50.00%,DELTA: 5.00

AO/DiOC6(3)

(a)

CODE NR. 304642.,PART: 21332.,MAX: 7.
M=100%,*=>MAX.BER.0- 50.00%,DELTA: 5.00

UNSTAINED
CONTROL

(b)

FIG.7

T349AC

○ ENTZ
▲ TUH
+ THERAP.IND.

1=ADRIBLAST. 10UG/ML
2=ENDOXAN 140UG/ML
3=CISPLATIN 10UG/ML
4=METHOTREXAT 10UG/ML
5=FLUORURAC. 150UG/ML
6=VELBE 1UG/ML
7=DACTINCMYC. 1UG/ML
8=VINCRIST. 100NG/ML
9=ACLACINCMY.16UG/ML
10,11=KONTR.UNBEH.
INKUB. 7D. 37C

LYMPHKNOTENMETASTASE

MAMMAKARZINOM

0106339

# FIG. 3

CODE NR. 304637.,PART: 47957.,MAX: 2758.
M=100%,==>MAX,BER.0- 50.00%,DELTA: 5.00

a.) QUINACRINE

CODE NR. 304639.,PART: 54054.,MAX: 2967.
M=100%,==>MAX,BER.0- 50.00%,DELTA: 5.00

b.) FLURAM

CODE NR. 304648.,PART: 74995.,MAX: 7071.
M=100%,==>MAX,BER.0- 100.00%,DELTA: 10.00

c.) ANS

CODE NR. 304647.,PART: 43562.,MAX: 1839.
M=100%,==>MAX,BER.0- 50.00%,DELTA: 5.00

d.) o-PHTHALALDEHYDE

0106339

# FIG.4

a.) 4-AMINO-ACRIDINE

b.) F-MALEIMIDE

c.) FDA

d.) ADB

# FIG.5

AO/DiOC6 (3) Anfärbung von Humanblutzellen

(1% Konturlinie)

# FIG. 6